# FASCICULE DE BREVET EUROPEEN

(11) **EP 1 060 739 B1**
(45) Date de publication et mention de la délivrance du brevet: **03.04.2013**
(21) Numéro de dépôt: 00480049.6
(22) Date de dépôt: 06.06.2000
(51) Int. Cl.: A61K 8/44, A61K 8/64, A61Q 19/06

(54) **Composition cosmétique amincissante à base de L-arginine ou d'un de ses dérivés**
Schlankmachendes kosmetisches Mittel enthaltend L-Arginin oder einen Derivaten
Slimming cosmetic composition containing L-arginine or a derivative thereof

(30) Priorité: 16.06.1999 FR 9907631
(43) Date de publication de la demande: 20.12.2000
(73) Titulaire: EXSYMOL S.A.M., 98000 Monte Carlo (MC)
(72) Inventeur: Seguin, Marie-Christine, MC 98000 (MC)
(74) Mandataire: Richaud, Fabien

(56) Documents cités:
- EP-A1- 1 060 739
- EP-A2- 1 046 392
- WO-A1-00/40217
- DE-A- 4 341 001
- FR-A- 2 745 498
- FR-A- 2 758 724
- FR-M- 958
- GB-A- 2 199 243

## Description

### Domaine technique :

La présente invention concerne une nouvelle composition cosmétique pour l'amincissement à base de L-arginine, d'un analogue de L-arginine, ou d'un de leurs dérivés, se présentant sous une forme applicable par voie topique.

### Etat de la technique :

L'industrie cosmétique est en permanence à la recherche de nouveaux actifs toujours plus performants et réellement efficaces pour lutter contre les surcharges adipeuses localisées.

Le tissu adipeux est constitué essentiellement d'adipocytes, cellules métaboliquement très actives assurant le maintien de l'équilibre énergétique au sein de l'organisme grâce à deux voies métaboliques antagonistes :
- la lipogenèse c'est à dire la biosynthèse, à partir de glucose et d'acides gras, de triglycérides stockés ensuite au sein des adipocytes
- la lipolyse qui consiste en l'hydrolyse des triglycérides en glycérol et acides gras libres.

Parmi les produits cosmétiques amincissants proposés dans le commerce, on trouve des compositions agissant par inhibition de la lipogenèse qui contiennent des inhibiteurs de la captation ou de la fixation du glucose nécessaire à la synthèse des triglycérides.

D'autres produits amincissants visent à favoriser la lipolyse en intervenant sur le taux d'AMPc qui stimule la lipase permettant l'hydrolyse des triglycérides ; la plupart de ces produits contiennent des bases xanthiques, particulièrement la caféine, souvent associées à des extraits végétaux pouvant renforcer leur activité.

Certains produits contiennent aussi des actifs agissant sur les récepteurs adrénergiques α et β qui inhibent et stimulent respectivement la lipolyse.

On a également mis en évidence l'influence des hormones sexuelles et des catécholamines sur les récepteurs β, mais leur usage est interdit en cosmétique.

Des progrès récents dans la compréhension des mécanismes de régulation de l'adipocyte permettent d'envisager de nouvelles stratégies cosmétiques pour des produits amincissants à application topique.

La nouvelle approche proposée dans ce brevet est l'utilisation d'un actif lipolytique topique agissant sur l'adipocyte par libération d'un médiateur cellulaire.

Une équipe de chercheurs a récemment identifié un régulateur potentiel de la lipolyse : le radical monoxyde d'azote (NO°) ou une forme redox associée (Modulation of White Adipose Tissue Lipolysis by Nitric Oxide, Gaudiot et Coll., J. Biol.Chem., 1998, 273, 13475-13481).

Il résulte de ces travaux que le radical NO° peut, dans un environnement redox approprié, fonctionner comme un facteur de stimulation de la lipolyse.

Cependant, l'utilisation du monoxyde d'azote ou d'un donneur de NO° n'est pas compatible avec une utilisation cosmétique.

En effet, le monoxyde d'azote a des propriétés physiologiques multiples car c'est un médiateur cellulaire agissant notamment au niveau des systèmes vasculaire et neuronal.

NO° est également une toxine cellulaire potentielle impliquée dans la réponse immunitaire. Au niveau de la peau, NO° participe à la réponse inflammatoire (notamment lors d'un stress photo-induit), module l'activité mélanocytaire, ou la prolifération cellulaire lors du processus de cicatrisation.

De plus, dans certaines conditions, le monoxyde d'azote peut générer *in vivo* des sous produits toxiques tels que l'anion peroxynitreux.

### Exposé de l'invention :

C'est pourquoi, un des buts de la présente invention est de résoudre une partie des problèmes évoqués ci-dessus en proposant une composition cosmétique pour l'amincissement contenant un actif cosmétique susceptible de générer le monoxyde d'azote de façon endogène.

La L-arginine est le substrat naturel de la NO-synthase, enzyme qui métabolise l'arginine pour libérer le monoxyde d'azote. Cette enzyme est présente naturellement au sein du tissu adipeux et permet de maintenir un taux basal de NO°.

Il a été prouvé qu'un apport extracellulaire de L-arginine entraînait une production de NO°.

Bien que cette enzyme soit très spécifique, il a été démontré récemment qu'elle pouvait accepter d'autres substrats, et notamment un composé analogue de la L-arginine, l'homo-L-arginine.

Dans le cadre de cette nouvelle approche cosmétique, l'application topique de l'actif a aussi été prise en compte, ce qui soulève la question de sa biodisponibilité c'est à dire sa capacité à agir sur sa cible biologique principale : l'adipocyte.

Ce type de cellule n'est effectivement pas localisé à proximité immédiate du site d'application de l'actif, mais dans une couche profonde de la peau, l'hypoderme.

C'est pourquoi un but important de l'invention est d'augmenter la biodisponibilité de l'actif cosmétique selon l'invention.

Une des caractéristiques de la composition cosmétique selon l'invention est donc de réduire la dégradation (par les arginases) ou la métabolisation de la L-arginine (ou de son analogue) par les couches supérieures de la peau, ou encore d'améliorer la pénétration cutanée de la L-arginine ou de son analogue.

La NO-synthase est également présente dans les cellules des couches supérieures de la peau telles que les kératinocytes et les fibroblastes.

Une autre caractéristique de l'invention est donc également d'éviter d'éventuels effets secondaires indésirables pouvant résulter de la production de NO° par les couches supérieures de la peau.

Une conséquence positive de la présence de NO-synthase dans les couches supérieures de la peau est que la composition lipolytique à base de précurseur de NO° sera capable de produire un signal lipolytique qui pourra être transmis vers une couche plus profonde, telle que l'hypoderme.

De plus, les cellules endothéliales, qui forment la paroi des microvaisseaux irriguant le derme et l'hypoderme, possèdent également des NO-synthases et peuvent donc participer à l'effet lipolytique.

Elles peuvent également contribuer à la diminution des surcharges adipeuses par un mécanisme particulier : le NO produit peut en effet induire une vasodilatation qui va favoriser le drainage du tissu adipeux.

Enfin, un mode d'action possible du radical NO°, suggéré par des résultats expérimentaux très récents, pourrait provenir de sa capacité à s'opposer à la multiplication des préadipocytes, des cellules précurseurs qui vont se différencier en adipocytes matures capables de stocker les graisses. Cette propriété pourrait ainsi s'opposer très efficacement à l'augmentation du tissu adipeux.

L'objet de la présente invention est donc de proposer l'utilisation de la L-arginine, d'un analogue de la L-arginine, ou d'un de leurs dérivés (ou l'un quelconque de leurs sels) agissant comme précurseur endogène de NO°, dans des compositions cosmétiques pour l'amincissement administrables par voie topique.

### Description détaillée de l'invention :

Jusqu'à ce jour, aucune utilisation de la L-arginine, ou d'un analogue de L-arginine précurseur de NO°, ou d'un de leurs dérivés, n'a été revendiquée pour l'amincissement.

Le brevet FR-A-2.758.724 propose une composition destinée à lutter contre la cellulite et les surcharges adipeuses à base de protamine, protéine contenant entre autre de l'arginine. La protamine aurait une action inhibitrice de la captation des acides gras libres par l'adipocyte dans le tissu adipeux sous-cutané. La protamine agirait donc par inhibition de la lipogenèse, ce qui n'est pas l'effet revendiqué dans la présente invention.

L'arginine est également utilisée dans le brevet FR-A-2.745.498 qui décrit une composition thérapeutique associant de l'arginine et du silicium utilisée dans le cadre d'un régime hyperprotéiné hypocalorique. Cette composition administrable par voie orale a pour but d'améliorer l'efficacité d'une diète protéinée grâce à l'activité lipolytique du silicium et d'en éviter les effets secondaires sur la fonction rénale grâce à l'arginine.

Le brevet EP-A-0.281.435 décrit un produit thérapeutique consistant en un complexe de silanol et d'acide aminé, ledit produit convenant à la préparation de médicaments régulateurs et activateurs du métabolisme, de la croissance et de la multiplication de certaines cellules. L'agent actif est le silanol, dont l'activité biologique est améliorée par la complexation avec un acide aminé naturel pouvant être la L-arginine.

La demande de brevet WO 2000/40217 A a été déposée le 07.01.2000, revendique la priorité du 08.01.1999 et a été publiée le 13.07.2000. Cette demande, qui divulgue des compositions à usage topique comprenant notamment de la N-acétyl-arginine en vue d'agir sur les désordres de la peau, est entrée en phase régionale européenne sous la référence EP1143925. Par conséquent, le contenu de la demande de brevet WO 2000/40217 telle que déposée est considéré comme compris dans l'état de la technique au sens de l'Article 54 (3) et (4) CBE 1973, sous réserve que la priorité revendiquée par ladite demande soit valablement reconnue.

Enfin, à notre connaissance, l'utilisation de la L-arginine comme précurseur de NO° a déjà été décrite (Cordeiro P.G., Santamaria E, Hu Q.Y. "Use of a nitric oxide precursor to protect pig myocutaneous flaps from ischemia-reperfusion injury" - Plastic & Reconstructive surgery, vol. 102, (1998) N°6, pp. 2040-2048), mais il s'agissait de protéger les tissus du stress oxydatif.

La présente invention a donc pour objet une composition topique cosmétique pour l'amincissement comprenant, en association avec tout excipient cosmétiquement approprié, au moins un composé qui est un analogue de la L-arginine ou un de ses dérivés, un dérivé de la L-arginine, ou l'un quelconque de leurs sels, ledit composé ayant la formule générale suivante : 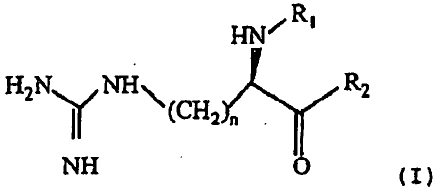 dans laquelle :
R1 représente un groupement hydroxyle, un radical acyle, acyloxy ou un aminoacide substitué ou non sur sa fonction α-aminée libre, lié par une liaison peptidique,
R2 représente un groupement hydroxyle, un radical amine, alkylamine ou alcoxy, un groupement silyloxy ou un aminoacide substitué ou non sur sa fonction α-carboxylique libre, lié par une liaison peptidique,
et n est égal à 3 ou 4
ledit composé étant différent de la N-acétyl-arginine et de la N-acétyl-homoarginine.

Un autre objet de l'invention est une composition topique cosmétique pour l'amincissement caractérisée en ce qu'elle comprend, en association avec tout excipient cosmétiquement approprié, au moins un composé qui est un analogue de L-arginine ou un de ses dérivés, un dérivé de la L-arginine, ou l'un quelconque de leurs sels, ledit composé ayant la formule générale (I) suivante : 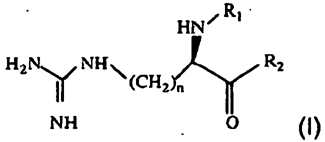 dans laquelle :
R₁ représente un groupement hydroxyle, un radical acyle, acyloxy ou
un aminoacide substitué ou non sur sa fonction α-aminée libre, lié par une liaison peptidique,
R₂ représente un radical alcoxy, amine, alkylamine, un groupement silyloxy ou un aminoacide substitué ou non sur sa fonction α-carboxylique, lié par une liaison peptidique,
et n = 3 ou 4.

Il est à noter que les radicaux R1 et R2 sont des substituants biodégradables des fonctions α-amine ou α-carboxylique de la L-arginine, ou de son analogue, pouvant être hydrolysés in vivo pour reformer l'aminoacide.

La L-arginine étant le substrat naturel de la NO-synthase, il est essentiel que, dans tous les composés selon l'invention, la stéréochimie du carbone asymétrique de la L-arginine soit respectée, afin que ces composés soient reconnus comme substrats par la NO-synthase.

Les composés de formule (I) préférés sont ceux pour lesquels R1 représente un atome d'hydrogène et R2 représente un groupement silyloxy.

De préférence, le groupement silyloxy a la formule générale suivante : 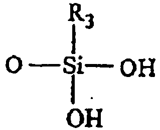 où R₃ représente un groupement alkyle.

Dans ce cas, une partie des silanols utilisés pour la synthèse du composé (I) peut se complexer ou se combiner par des liaisons faibles avec le groupement NH₂ de la L-arginine (ou de son analogue), ce qui contribue à augmenter la stabilité du composé (I) et à éviter sa métabolisation dans les couches supérieures de la peau.

Parmi ces composés, un composé préféré est le monométhyl silanetriol de L- arginine.

Un mode de réalisation particulièrement avantageux de l'invention consiste à réduire temporairement la polarité de la L-arginine ou de son analogue, de façon à favoriser la pénétration de l'actif dans l'épiderme.

Ceci est le plus simplement réalisé par estérification de la fonction α-carboxylique et/ou amidification de la fonction α-aminée, ce qui rend ces fonctions non ionisables à pH physiologique.

La substitution des fonctions α-carboxylique et α-aminée par des radicaux eux-mêmes hydrophobes contribue à renforcer le caractère lipophile de la molécule et à favoriser sa pénétration cutanée.

La biodégradabilité des substituants, c'est à dire leur capacité à s'hydrolyser aux conditions de pH physiologiques ou en présence d'enzymes cutanés, est un élément important de l'invention car les composés substitués en position alpha ne sont pas biologiquement actifs.

De préférence, la fonction α-aminée est substituée par un radical acétyle ou un substituant plus hydrophobe tel qu'un radical tert-butyloxycarbonyle (Boc).

De préférence, la fonction α-carboxylique est substituée par un radical R2 alcoxy ou alkylamine porteur d'un groupement éthyle ou d'un substituant plus hydrophobe tel que le radical *n*-butyle, *n*-octyle ou encore par le pivalloyloxyméthyle, un substituant qui a la particularité d'être stable au pH externe de la peau (pH 5 environ) et hydrolysé à pH proche de la neutralité, ce qui est le pH des couches plus profondes de la peau.

Suivant encore un autre mode de réalisation préféré de l'invention, le composé de formule générale (I) est tel que R₁ et/ou R₂ est un aminoacide choisi parmi les aminoacides suivants : alanine, cystéine, glycine, hydroxyproline, leucine, isoleucine, méthionine, phénylalanine, proline, sérine, thréonine, tryptophane, valine, asparagine, acide glutamique, acide pyro-glutamique, acide aspartique, glutamine, arginine, histidine, lysine.

De préférence, l'aminoacide est l'alanine, la leucine, l'isoleucine, la phénylalanine, le tryptophane ou la valine qui sont des aminoacides hydrophobes permettant de renforcer la lipophilie du composé, afin d'augmenter son affinité pour la peau.

Lorsque le radical R₂ est un aminoacide, il est préférable de substituer sa fonction α-carboxylique libre et/ou la fonction α-aminée libre de l'aminoacide R₁ de façon à rendre cette fonction non ionisable à pH physiologique.

De préférence, ces fonctions sont substituées par les groupements cités précédemment.

Comme on l'a dit, un but important de l'invention est de proposer un précurseur de NO°, la L-arginine ou son analogue, dont la biodisponibilité est compatible avec une action sur l'hypoderme, une couche profonde de la peau.

Il est donc utile de réaliser un composé ayant une forme propre à éviter qu'il ne soit métabolisé dans les couches supérieures de la peau avant d'avoir atteint l'hypoderme.

Ceci est obtenu en première intention lors de la substitution des fonctions α-carboxylique et α-aminée de la L-arginine. En effet, ces formes sont biologiquement inactives (concept de prodrogue) et n'entreront donc pas dans les voies métaboliques utilisant cet aminoacide.

Ces formes ne sont notamment pas substrat des NO-synthases (Hrabak A, Bajor T, Temesi A "Comparison of substrate and inhibitor specificity of arginase and nitric oxyde (NO) synthase for arginine analogues and related compounds in murine and rat macrophages" - Biochem. Biophys. Res. Commun., vol. 198, (1994) pp. 206-212).

Cette résistance à la "désactivation enzymatique" par les couches supérieures de la peau sera encore renforcée lorsque le substituant R₁ et/ou le substituant R₂ porte un radical stériquement encombrant.

Ainsi, lorsque R₁ est un radical acyloxy et/ou R₂ est un radical alcoxy ou alkylamine, la chaîne alkyle portée par ces radicaux peut être un groupement isopropyle, isobutyle, secbutyle ou tertiobutyle.

De même, lorsque R₁ et/ou R₂ est un aminoacide, celui-ci est choisi parmi les aminoacides possédant une chaîne latérale encombrante tels que la valine, la leucine ou l'isoleucine.

Selon un autre mode de réalisation permettant d'augmenter la résistance à la dégradation enzymatique du composé (I), les radicaux R₁ et/ou R₂ sont choisis parmi des acides aminés substrats d'enzymes spécifiques de manière à rendre ledit composé moins sensible à la désactivation enzymatique lors de son passage dans les couches supérieures de la peau.

De tels acides aminés peuvent être la citrulline, l'homosérine, la norvaline, l'ornithine, la pénicillamine ou la sarcosine. Selon ce mode de réalisation, un composé particulièrement intéressant est le dipeptide naturel L-citrullinyl-L-arginine (R₁ = citrulline et R₂ = OH) produit par une algue rouge (*Chondrus crispus*).

Un objectif particulièrement important de l'invention est de proposer un précurseur de NO° qui ne provoque pas d'effet secondaire gênant, ce qui est inacceptable pour un actif cosmétique.

Ainsi, comme on l'a précisé, la composition cosmétique objet de l'invention est conçue de façon à éviter la production de NO° par les cellules des couches supérieures de la peau.

Les kératinocytes ou les fibroblastes ne possèdent normalement qu'une forme de NO-synthase dite "constitutive" qui ne produit le NO° qu'en très faibles quantités (quantités compatibles avec le rôle de messager cellulaire de NO°), et uniquement en réponse à des stimuli très spécifiques.

Toutefois ces cellules, dans certaines conditions pathologiques (inflammation notamment), peuvent exprimer des formes dites "inductibles" de NO-synthases à même de produire des quantités plus importantes de NO°.

Dans ce contexte un mode de réalisation particulier de l'invention vise à s'opposer à un sous-produit toxique du radical NO°, l'acide peroxynitreux (HOONO), qui peut être produit lorsque la peau est soumise à un stress oxydatif et dont la toxicité est renforcée en présence de métaux de transition.

Le principe consiste à condenser le précurseur de NO° sur une molécule à même de s'opposer à la toxicité de l'acide peroxynitreux, qui sera libérée lors de la "bioactivation" par les enzymes cutanés.

Selon ce mode de réalisation particulier de l'invention, on condense sur la L-arginine ou son analogue un substituant R₁ ou R₂ biodégradable possédant une fonction thiol (SH) ou thioéther. En effet, l'atome de soufre confère la possibilité de fixer les ions métalliques qui participent à la formation d'espèces cytotoxiques dérivées du radical NO° , et par conséquent de les neutraliser.

On sait par ailleurs qu'*in vivo* les aminoacides possédant un groupement thiol, et en particulier la cystéine, constituent une cible privilégiée de l'acide peroxynitreux.

On condensera donc avantageusement sur la L-arginine ou son analogue les aminoacides possédant une fonction thiol choisis parmi la L-cystéine,la N-acétyl-cystéine la L-méthionine ou la pénicillamine .

D'autres acides aminés conviennent également pour éviter d'éventuels effets secondaires dus à la formation d'acide peroxynitreux, et en particulier la L-histidine qui possède un noyau imidazole lui conférant la propriété de piéger les radicaux libres pouvant être générés par l'acide peroxynitreux (décomposition homolytique pour former OH° et NO°). De plus, L'histidine est connue pour ses propriétés de chélation des métaux de transition qui catalysent les réactions toxiques associées à l'acide peroxynitreux.

On peut aussi choisir avantageusement l'acide glutamique ou l'acide aspartique dont les fonctions α-carboxyliques confèrent des propriétés de chélations des métaux de transition.

Les compositions cosmétiques selon l'invention peuvent avantageusement contenir au moins un composé aux propriétés anti-oxydantes de façon à neutraliser les radicaux libres pouvant être générés par l'acide peroxynitreux, et à réparer les dommages causés par cette espèce (agent peroxydant des membranes cellulaires).

De préférence, ce composé anti-oxydant est un pseudodipeptide du type de ceux obtenus par couplage de l'histamine et d'un acide aminé, commercialisés par la demanderesse et décrits dans la demande de brevet internationale WO94/19325.

Un mode particulier de réalisation de l'invention consiste à obtenir une composition contenant un précurseur permettant la synthèse endogène de NO° tout en modifiant favorablement le "statut redox" du tissu adipeux.

En effet, selon Gaudiot et coll., l'activité lipolytique du monoxyde d'azote est modulée par son état redox (NO°, NO⁺ ou NO⁻), il est particulièrement approprié de créer un état redox (tissulaire ou intracellulaire) qui va optimiser l'activité lipolytique de ce messager.

Ceci peut être obtenu en associant au composé de formule générale (I) un anti-oxydant tel que la vitamine E (et autres tocophérols apparentés), la vitamine C (acide ascorbique et dérivés) ou un pseudodipeptide du type de ceux mentionnés précédemment.

On peut aussi avantageusement condenser sur la L-arginine ou son analogue un composé présentant des propriétés antioxydantes. Certains de ces composés tels que les aminoacides ont déjà été mentionnés mais la cystéine et préférablement la N-acétyl-cystéine sont particulièrement indiqués dans la mesure où leur activité sur le statut redox des cellules a été décrit.

D'autre part, lorsque le composé de formule générale (I) est le monométhylsilanetriol de L-arginine, le monométhyl silanetriol produit lors de l'hydrolyse du composé, peut lui aussi moduler le statut redox cellulaire de façon à potentialiser l'effet lipolytique.

Enfin, dans la mesure où l'objet de l'invention consiste à apporter à la peau un substrat de la NO-synthase propre à induire la formation endogène de NO, tout additif favorisant l'activité de cette enzyme pourra s'inscrire dans cette approche.

Il a été démontré que les composés portant une fonction thiol réduite étaient nécessaires pour une activité maximale de l'enzyme.

C'est pourquoi la cystéine et d'autres composés comprenant une fonction thiol seront des additifs préférés.

On peut citer le NADPH, un cofacteur nécessaire à l'activité de l'enzyme, qui est consommé lors de la formation du NO.

Toute substance pouvant contribuer à augmenter le taux de NADPH dans les cellules cutanées pourra donc favoriser un effet lipolytique NO-dépendant.

De même, le calcium ou toute substance pouvant augmenter le taux de calcium intracellulaire (cet élément est également un cofacteur de l'enzyme).

A ce titre, le monométhyl silanetriol de L-arginine est un des composés indiqués, dans la mesure où le monométhyl silanetriol peut favoriser l'apport de calcium aux cellules (traitement de l'ostéoporose).

Les composés de formule générale (I) peuvent être préparés suivant des méthodes connues de l'homme du métier.

Les compositions cosmétiques selon l'invention peuvent être utilisées sous différentes formes compatibles en cosmétique c'est à dire sous forme de solution, d'émulsion ou de dispersion, sous forme de liposomes, microparticules ou nanoparticules ou encore sur des matrices de polymères organiques, de silice ou autres supports minéraux.

On choisira de préférence des formes propres à favoriser la pénétration intradermale telles que les microémulsions, les liposomes ou encore l'Emulzome^{®}, microdispersion fluide de corps gras/eau commercialisée par la demanderesse, qui présente une bioaffinité élevée pour la peau.

Les compositions selon l'invention peuvent se présenter sous toute forme cosmétiquement appropriée, et notamment sous toutes les formes appropriées à un usage topique : crème, lait, lotion, gel, spray, patch.

Tous les additifs et excipients couramment utilisés dans l'industrie cosmétique peuvent être employés pour réaliser l'invention pourvu qu'ils soient compatibles avec le composé de formule générale (I).

Parmi ces additifs et excipients, on peut citer le Transcutol^{®} (éther monométhylique de diéthylène glycol), l'a-Bisabolol, l'acide oléique ou encore l'urée qui, en plus de leurs propriétés spécifiques, favorisent la pénétration cutanée.

Les exemples qui suivent illustrent de façon non limitative les compositions cosmétiques selon l'invention.

### Exemples de structures :

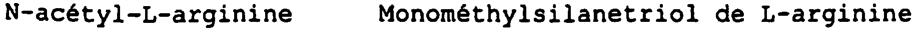

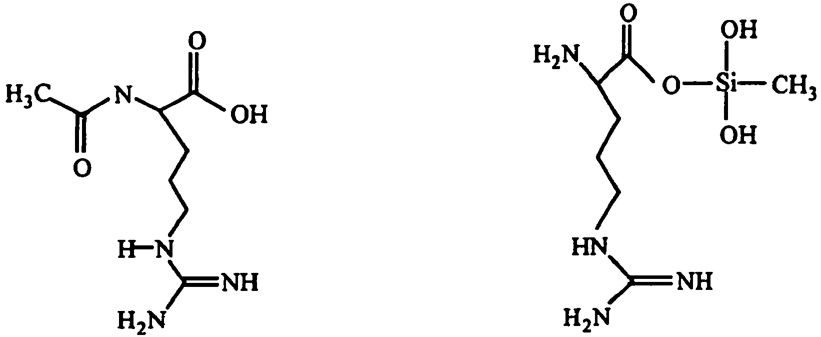

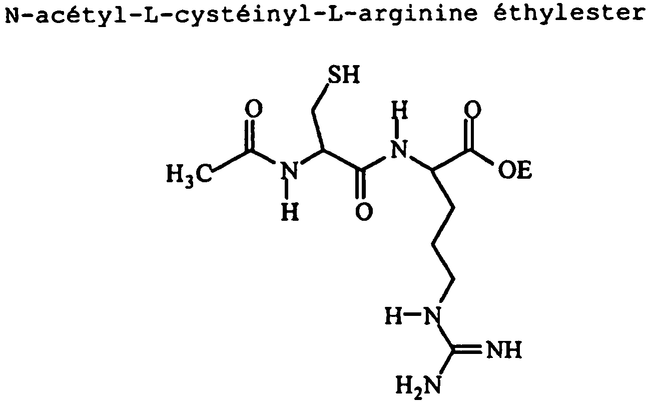

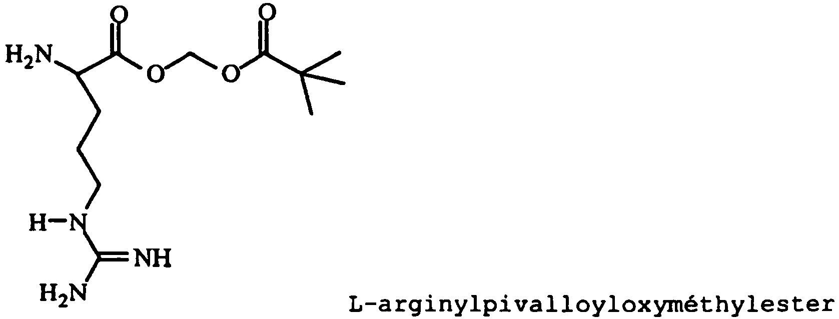

### Exemple Emulsion fluide (microdispersion huile dans l'eau)

| | | |
|---|---|---|
| Sepigel 305^{®} (Polyacrylamide, C13-14 isoparaffine, Laureth-7) | | 4,00 g |
| triglycérides d'acides caprique et caprilique | | 5,00 g |
| myristate d'isopropyle | | 5,00 g |
| diheptanoate de néopenthyl glycol | | 2,00 g |
| glycérine ( glycérol) | | 5,00 g |
| polyisobutène hydrogéné | | 2,15 g |
| cyclomethicone | | 0,60 g |
| heptanoate de stéaryle | | 1,43 g |
| imidazolidinyl urée | | 0,30 g |
| phénoxyéthanol | | 0,20 g |
| Monométhylsilanetriol de L-arginine | | 4,00 g |
| ALISTIN^{®} (solution hydroglycolique de carcinine chlorhydrate à 10%) | | 0,20 g |
| α-bisabolol | | 0,50 g |
| EDTA disodique | | 0,10 g |
| Eau | qsp | 100,00 g |

### Exemple 2 :Gel glycolique

| | | |
|---|---|---|
| Carbomer | | 0,50 g |
| Transcutol^{®} (éthoxydiglycol) | | 2,00 g |
| Propylène glycol | | 1,00 g |
| Sorbitol | | 1,00 g |
| Oleth-20 | | 1,50 g |
| Phénoxy éthanol | | 0,50 g |
| 6N-acétyl-L-arginine | | 5 g |
| Solution hydroglycolique de L-pyroglutamylhistamine à 10% | | 0,20 g |
| urée | | 3,00 g |
| EDTA disodique | | 0,10 g |
| Hydroxyde de sodium | | 0,10 g |
| Eau | qsp | 100,00 g |

### Exemple Crème amincissante

| | | |
|---|---|---|
| Sepigel 305^{®} (Polyacrylamide, C₁₃₋₁₄ isoparaffin, Laureth 7) | | 2,00 g |
| imidazolidinyl urée | | 0,30 g |
| parahydroxybenzoate de méthyle sodé | | 0,10 g |
| parahydroxybenzoate de propyle | | 0,05 g |
| N-acétyl-L-cystéinyl-L-arginine éthylester | | 4,70 g |
| Tegocare 150 (stéarate de glycéryle, | | 10 g |
| stéareth-25, céteth-20, alcool stéarylique) | | |
| octanoate de cétéaryle | | 10,00 g |
| huile de macadamia | | 10,00 g |
| diméthicone copolyol | | 0,30 g |
| diméthicone | | 0,20 g |
| glycérine | | 3,00 g |
| acide oléique | | 1,00 g |
| Eau | qsp | 100,00 g |

### Exemple 4: Lait amincissant

| | | |
|---|---|---|
| Sepigel 501^{®} (copolymères d'acrylamide, huile de paraffine, polysorbate-85) | | 2,00 g |
| isononanoate d'octyle | | 2,00 g |
| diméthicone copolyol | | 0,50 g |
| cétéaryl glucoside | | 0,80 g |
| polyisobutène hydrogéné | | 0,54 g |
| cyclométhicone | | 0,15 g |
| heptanoate de stéaryle | | 0,36 g |
| imidazolidinyl urée | | 0,20 g |
| phénoxyéthanol | | 0,30 g |
| L-arginylpivalloyloxyméthylester | | 3,50 g |
| urée | | 1,50 g |
| Eau | qsp | 100,00 g |

### Exemple 5 Etude comparative de la diffusion cutanée

Des rats hairless sont sacrifiés par injection intracardiaque de pentobarbital ; la peau ventrale est récupérée et l'hypoderme est enlevé au scalpel.

0,2 ml par cm² de solution à tester sont déposés sur un morceau de peau (derme et épiderme) qui est ensuite monté sur une cellule de diffusion. La diffusion est réalisée contre du tampon phosphate contenant 0,5 % de tween 20.

A différents temps, le tampon phosphate est remplacé et l'arginine est dosée par HPLC dans les fractions de dialysat récupérées.

| Produits testés (solution à 30 mM) | % de diffusion après 5h |
|---|---|
| L-Arginine | 15 |
| Monométhylsilanetriol de L-arginine | 42 |
| N-acétyl L-arginine | 47 |
| N-acétyl-L-cystéinyl-L-arginine méthylester | 61 |
| L-arginylpivalloyloxyméthylester | 51 |

### Exemple 6 : Activité lipolytique in vitro

On teste l'activité lipolytique *in vitro* de solutions contenant des composés selon l'invention à 4%.

Le témoin négatif est la solution qui sert à diluer les actifs (NH₄HCO₃ 0,01 M).

Le témoin positif est la caféine à 1,5 g/l.

Des segments de tissu adipeux périrénal de lapins mâles sont incubés dans un tampon Krebs-Ringer bicarbonate, pH 7,4, qui contient 5,5 mM glucose, 40 mg/ml de sérum albumine bovin. Le volume total d'incubation est de 3 ml avec les substances à tester préparées dans 0,01 M NH₄HCO₃ et additionné dans un volume de 0,1 ml. Les préparations sont placées sous agitation à 37°C, sous une atmosphère à 95 % O₂, 5 % CO₂.

Les taux d'activité lipolytiques sont quantifiés par la libération du glycérol dans le milieu d'incubation et sont exprimés par gramme de lipide. Le glycérol est dosé en duplicata par la méthode enzymatique : glycérokinase, glycérophosphate deshydrogénase (Boerhinger-Mannheim), méthode de Wieland (32), modifiée par Vaughan (33).

### Résultats:

| | Glycérol (nmoles/g de lipides) |
|---|---|
| Témoin | 0,75±0,3 |
| Monométhylsilanetriol de L-arginine 15 mg/l | 1±0,5 |
| Monométhylsilanetriol de L-arginine 30 mg/l | 2,40±0,35 |
| Monométhylsilanetriol de L-arginine 45 mg/l | 2,5±0,35 |
| Caféine 1,5g/l | 1,45±0,25 |

### Exemple 7: test in vivo sur rats

Trois lots de 4 rats ZUCKER *fa*/*fa* définis de manière homogène ont été intégrés dans l'animalerie à 8 semaines. L'expérimentation débute lorsque les rats ont atteint 9 semaines et des poids comparables (240 ± 15 g) ; ils sont placés en cage individuelle avec eau et granulés *ad libidum.* Une épilation est pratiquée circulairement sur l'ensemble des rats en partie médiane de manière à tatouer un axe entre le cou et la queue.

Sur un lot I, on applique quotidiennement sur la partie épilée l'émulsion définie dans l'exemple 1 jusqu'à absorption en enlevant tout excès.

Sur un lot II, une ionisation de salicylate d'arginium à 1% est pratiquée tous les deux jours sous 12 volts à raison de 0,006 mA/cm² pendant 7 minutes, 2 électrodes traitantes de 2,5 cm² étant fixées latéralement sur les flancs du rat et reliées au pôle positif.

Un 3ème lot sert de témoin (lot T).

Ces expérimentations sont menées sur 5 semaines.

Les résultats suivants montrent une différence significative dans l'augmentation de la masse adipeuse entre les rats traités et les rats témoins.

| Semaine | 1 | | 2 | | 3 | | 4 | | 5 | |
|---|---|---|---|---|---|---|---|---|---|---|
| | Poids g | Mens mm | Poids g | Mens mm | Poids g | Mens mm | Poids g | Mens mm | Poids g | Mens mm |
| Lot T | 275,5 | 187,25 | 315,75 | 214,25 | 347,25 | 237 | 375,5 | 255,75 | 402,5 | 273,25 |
| Lot I | 282 | 191,25 | 318,75 | 215 | 347,25 | 232,75 | 368,25 | 245,25 | 386,5 | 254,75 |
| Lot II | 280,25 | 189,75 | 318,25 | 213,25 | 345,5 | 224,25 | 367,25 | 233,25 | 386,25 | 238,5 |

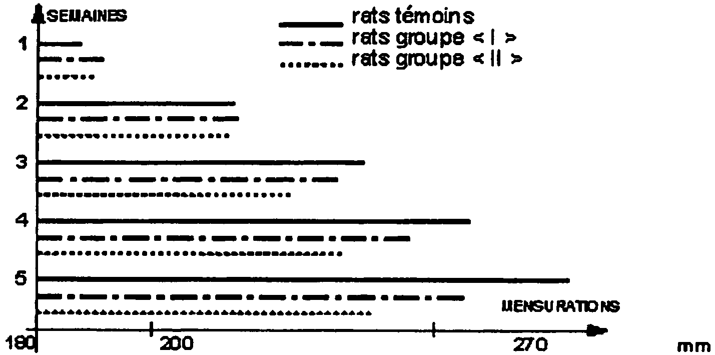

## Revendications

1. Composition topique cosmétique pour l'amincissement **caractérisée en ce qu'**elle comprend,
en association avec tout excipient cosmétiquement approprié, au moins un composé qui est un analogue de L-arginine ou un de ses dérivés, un dérivé de la L-arginine, ou l'un quelconque de leurs sels, ledit composé ayant la formule générale (I) suivante : 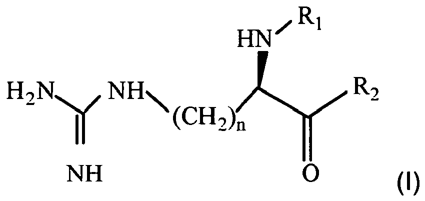 dans laquelle :
R₁ représente un groupement hydroxyle, un radical acyle, acyloxy ou un aminoacide substitué ou non sur sa fonction α-aminée libre, lié par une liaison peptidique,
R₂ représente un groupement hydroxyle, un radical alcoxy, amine, alkylamine, un groupement silyloxy ou un aminoacide substitué ou non sur sa fonction α-carboxylique, lié par une liaison peptidique,
et n = 3 ou 4
ledit composé étant différent de la N-acétyl-arginine et de la N-acétyl-homoarginine.

2. Composition topique cosmétique pour l'amincissement **caractérisée en ce qu'**elle comprend, en association avec tout excipient cosmétiquement approprié, au moins un composé qui est un analogue de L-arginine ou un de ses dérivés, un dérivé de la L-arginine, ou l'un quelconque de leurs sels, ledit composé ayant la formule générale (I)suivante : 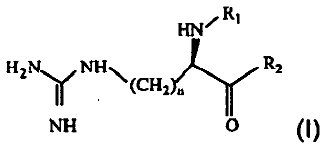 dans laquelle :
R₁ représente un groupement hydroxyle, un radical acyle, acyloxy ou un aminoacide substitué ou non sur sa fonction α-aminée libre, lié par une liaison peptidique,
R₂ représente un radical alcoxy, amine, alkylamine, un groupement silyloxy ou un aminoacide substitué ou non sur sa fonction α-carboxylique, lié par une liaison peptidique.
et n = 3 ou 4.

3. Composition cosmétique selon l'une des revendications 1 ou 2, **caractérisée en ce que** n = 3.

4. Composition cosmétique selon l'une des revendications 1, 2 ou 3, **caractérisée en ce que** R₂ représente un groupement silyloxy ayant la formule générale suivante: 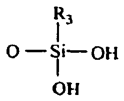 dans laquelle R₃ représente un groupement alkyle.

5. Composition cosmétique selon l'une quelconque des revendications 1 à 4, **caractérisée en ce que** ledit composé de formule (I) est le monométhyl silanetriol de L-arginine.

6. Composition cosmétique selon l'une des revendications 1, 2 ou 3, **caractérisée en ce que** ledit composé de formule (I) est tel que R₁ et/ou R₂ est un acide aminé substitué ou non sur sa fonction α-amine ou α-carboxylique, choisi parmi les aminoacides suivants : alanine, cystéine, glycine, hydroxyproline, isoleucine, leucine, méthionine, phénylalanine, proline, hydroxyproline, sérine, thréonine, tryptophane, asparagine, valine, acide aspartique, acide glutamique, acide pyroglutamique, glutamine, arginine, histidine, lysine.

7. Composition cosmétique selon la revendication 6, **caractérisée en ce que** ledit composé de formule générale (I) est tel R₁ et/ou R₂ est l'alanine, la leucine, l'isoleucine, la phénylalanine, le tryptophane ou la valine.

8. Composition cosmétique selon la revendication 6 **caractérisée en ce que** ledit composé de formule générale (I) est tel que R₁ et/ou R₂ est la méthionine, la cystéine, l'histidine, l'acide aspartique ou l'acide glutamique.

9. Composition cosmétique selon l'une des revendications 1, 2 ou 3, **caractérisée en ce que** ledit composé de formule (I) est tel que R₁ et/ou R₂ représente un acide aminé substitué ou non sur sa fonction α-amine ou α-carboxylique choisi parmi les aminoacides suivants : citrulline, homosérine, norvaline, ornithine, pénicillamine ou sarcosine.

10. Composition cosmétique selon l'une des revendications 1, 2 ou 3, **caractérisée en ce que** ledit composé de formule (I) est tel que R1 est un radical acyl ou acyloxy portant un groupement isopropyle, sec-butyle, tert-butyle, isobutyle et/ou R2 est un radical alcoxy ou alkylamine portant un groupement isopropyle, n-butyle, sec-butyle, tert-butyle, isobutyle, n-octyle ou pivalloyloxyméthyle.

11. Composition cosmétique selon l'une quelconque des revendications 1 à 10, **caractérisée en ce qu'**elle comprend en outre un antioxydant tel que la vitamine E, l'acide ascorbique ou leurs dérivés, ou un aminoacide possédant un groupement thiol tel que la N-acétyl-cystéine.

12. Composition cosmétique selon l'une quelconque des revendications 1 à 11, **caractérisée en ce qu'**elle comprend en outre un pseudodipeptide du type de ceux obtenus par couplage de l'histamine et d'un acide aminé.

13. Composition cosmétique selon l'une des revendications 1 à 12, **caractérisée en ce qu'**elle contient en outre un activateur de NO-synthase tel que le NADPH, le calcium ou toute substance propre à augmenter le taux de calcium intracellulaire.

14. Composition cosmétique selon l'une des revendications 1 à 13, **caractérisée en ce qu'**elle se présente sous toute forme couramment employée en cosmétique à savoir crème, lait, lotion, gel, patch ou spray.

15. Composition selon l'une des revendications précédentes, **caractérisée en ce que** ledit composé est un précurseur endogène de NO°.

16. Composition selon l'une des revendications précédentes, **caractérisée en ce qu'**elle est apte à agir sur un adipocyte par libération de NO°.

17. Utilisation d'au moins un composé qui est un analogue de L-arginine ou un de ses dérivés, un dérivé de la L-arginine, ou l'un quelconque de leurs sels, dans la préparation d'une composition pharmaceutique, en application topique, pour l'amincissement, par libération endogène de NO°, ledit composé ayant la formule générale (I) suivante: 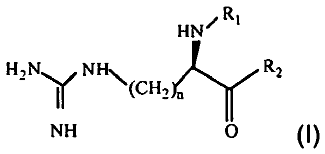 dans laquelle :
R₁ représente un atome d'hydrogène, un groupement hydroxyle, un radical acyle, acyloxy ou un aminoacide substitué ou non sur sa fonction α-aminée libre, lié par une liaison peptidique,
R₂ représente un radical alcoxy, amine, alkylamine, un groupement silyloxy ou un aminoacide substitué ou non sur sa fonction α-carboxylique, lié par une liaison peptidique,
et n = 3 ou 4.

## Claims

1. Topical cosmetic composition for slimming **characterized in that** it comprises, in combination with any cosmetically suitable excipient, at least one compound which is an analogue of L-arginine or a derivative thereof, a derivative of L-arginine, or any of their salts, said compound having the following general formula (I): 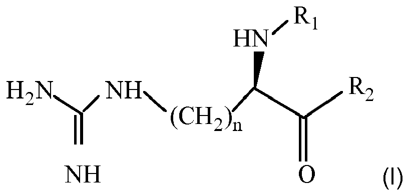 wherein :
R₁ represents a hydroxyl group, an acyl or acyloxy radical or an amino acid unsubstituted or substituted on its free α-amino function, bound by a peptide bond,
R₂ represents a hydroxyl group, an alkoxy, amine or alkylamine radical, a silyloxy group or an amino acid unsubstituted or substituted on its free α-carboxylic function, bound by a peptide bond,
and n = 3 or 4,
said derivative being different from N-acetyl-arginine and N-acetyl-homoarginine.

2. Topical cosmetic composition for slimming **characterized in that** it comprises, in combination with any cosmetically suitable excipient, at least one compound which is an analogue of L-arginine or a derivative thereof, a derivative of L-arginine, or any of their salts, said compound having the following general formula (I): 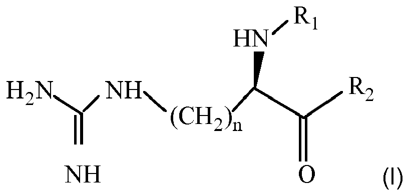 wherein :
R₁ represents a hydroxyl group, an acyl or acyloxy radical or an amino acid unsubstituted or substituted on its free α- amino function, bound by a peptide bond,
R₂ represents an alkoxy, amine or alkylamine radical, a silyloxy group or an amino acid unsubstituted or substituted on its free α-carboxylic function, bound by a peptide bond,
and n = 3 or 4.

3. Cosmetic composition according to one of claims 1 or 2, **characterized in that** n = 3.

4. Cosmetic composition according to one of claims 1, 2 or 3, **characterized in that** R₂ represents a silyloxy group having the following general formula: 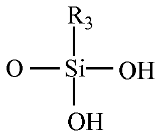 wherein R₃ represents an alkyl group.

5. Cosmetic composition according to one of claims 1 to 4, **characterized in that** said compound of formula (I) is the L-arginine monomethylsilanetriol.

6. Cosmetic composition according to one of claims 1, 2 or 3, **characterized in that** said compound of formula (I) is such that R₁ and/or R₂ is an amino acid unsubstituted or substituted on its free α-amino or α-carboxylic function, chosen among the following amino acids : alanine, cysteine, glycine, hydroxyproline, isoleucine, leucine, methionine, phenylalanine, proline, hydroxyproline, serine, threonine, tryptophan, asparagine, valine, aspartic acid, glutamic acid, pyroglutamic acid, glutamine, arginine, histidine, lysine.

7. Cosmetic composition according to claim 6, **characterized in that** said compound of general formula (I) is such that R₁ and/or R₂ is alanine, leucine, isoleucine, phenylalanine, tryptophan or valine.

8. Cosmetic composition according to claim 6 **characterized in that** said compound of general formula (I) is such that R₁ and/or R₂ is methionine, cysteine, histidine, aspartic acid or glutamic acid.

9. Cosmetic composition according to one of claims 1, 2 or 3, **characterized in that** said compound of formula (I) is such that R₁ and/or R₂ represents an amino acid unsubstituted or substituted on its α-amino or α-carboxylic function, chosen among the following amino acids : citrulline, homoserine, norvaline, ornithine, penicillamine or sarcosine.

10. Cosmetic composition according to one of claims 1, 2 or 3, **characterized in that** said compound of formula (I) is such that R₁ is an acyl or acyloxy radical bearing an isopropyl, sec-butyl, tert-butyl, isobutyl group and/or R₂ is an alkoxy or alkylamine radical bearing an isopropyl, n-butyl, sec-butyl, tert-butyl, isobutyl, n-octyl or pivalloyloxymethyl group.

11. Cosmetic composition according to one of claims 1 to 10, **characterized in that** it further comprises an antioxidant such as vitamin E, ascorbic acid or their derivatives, or a thiol-containing amino acid such as N-acetyl-cysteine.

12. Cosmetic composition according to one of claims 1 to 11, **characterized in that** it further comprises a pseudodipeptide of the type obtained by the coupling between histamine and an amino acid.

13. Cosmetic composition according to one of claims 1 to 12, **characterized in that** it further comprises an activator of NO-synthase such as NADPH, calcium or any substance able to increase intracellular calcium content.

14. Cosmetic composition according to one of claims 1 to 13, **characterized in that** it is under any form commonly used in cosmetics namely cream, milk, lotion, gel, patch or spray.

15. Cosmetic composition according to one of previous claims, **characterized in that** said compound is an endogenous precursor of NO°.

16. Cosmetic composition according to one of previous claims, **characterized in that** it is capable of acting on an adipocyte by release of NO°.

17. Use of at least one compound which is an analogue of L-arginine or a derivative thereof, a derivative of L-arginine, or any of their salts, in the manufacture of a pharmaceutical composition, by topical application, for slimming, by endogenous release of NO°, said compound having the following general formula (I) : 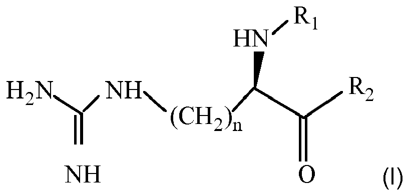 wherein :
R₁ represents a hydrogen atom, a hydroxyl group, an acyl or acyloxy radical or an amino acid unsubstituted or substituted on its free α-amino function, bound by a peptide bond,
R₂ represents an alkoxy, amine, alkylamine radical, a silyloxy group or an amino acid unsubstituted or substituted on its free α-carboxylic function, bound by a peptide bond,
and n = 3 or 4.

## Patentansprüche

1. Topische kosmetische Zusammensetzung mit schlankmachender Wirkung, **dadurch gekennzeichnet, dass** sie verbunden mit jedem kosmetisch geeigneten Trägerstoff mindestens eine Verbindung umfasst, die ein Analogstoff von L-Arginin oder einer seiner Derivate, ein Derivat des L-Arginins oder irgendeines ihrer Salze ist, wobei die Verbindung die folgende allgemeine Formel (I) aufweist: 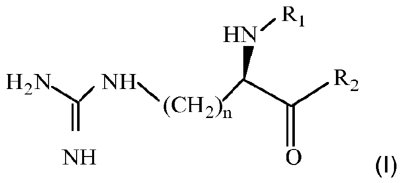 in der:
R₁ eine Hydroxylgruppe, ein Acyl-, Acyloxyradikal oder eine Aminosäure darstellt, deren freie funktionelle α-Aminogruppe substituiert ist oder nicht, gebunden durch eine Peptidbindung,
R₂ eine Hydroxylgruppe, ein Alkoxy-, Amino-, Alkylaminoradikal, eine Silyloxygruppe oder eine Aminosäure darstellt, deren funktionelle α-Carboxylgruppe substituiert ist oder nicht, gebunden durch eine Peptidbindung,
und n = 3 oder 4 ist,
wobei sich die Verbindung von dem N-Acetyl-arginin und dem N-Acetyl-homoarginin unterscheidet.

2. Topische kosmetische Zusammensetzung mit schlankmachender Wirkung, **dadurch gekennzeichnet, dass** sie verbunden mit jedem kosmetisch geeigneten Trägerstoff mindestens eine Verbindung umfasst, die ein Analogstoff von L-Arginin oder einer seiner Derivate, ein Derivat des L-Arginins oder irgendeines ihrer Salze ist, wobei die Verbindung die folgende allgemeine Formel (I) aufweist: 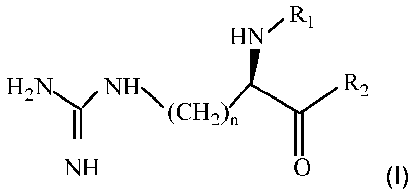 in der:
R₁ eine Hydroxylgruppe, ein Acyl-, Acyloxyradikal oder eine Aminosäure darstellt, deren freie funktionelle α-Aminogruppe substituiert ist oder nicht, gebunden durch eine Peptidbindung,
R₂ ein Alkoxy-, Amino-, Alkylaminoradikal, eine Silyloxygruppe oder eine Aminosäure darstellt, deren funktionelle α-Carboxylgruppe substituiert ist oder nicht, gebunden durch eine Peptidbindung,
und n = 3 oder 4 ist.

3. Kosmetische Zusammensetzung gemäß einem der Ansprüche 1 oder 2, **dadurch gekennzeichnet, dass** n = 3 ist.

4. Kosmetische Zusammensetzung gemäß einem der Ansprüche 1, 2 oder 3, **dadurch gekennzeichnet, dass** R₂ eine Silyloxygruppe darstellt, die die folgende allgemeine Formel aufweist: 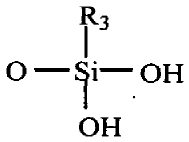 in der R₃ eine Alkylgruppe darstellt.

5. Kosmetische Zusammensetzung gemäß irgendeinem der Ansprüche 1 bis 4, **dadurch gekennzeichnet, dass** die Verbindung mit der Formel (I) L-Arginin-Monomethylsilantriol ist.

6. Kosmetische Zusammensetzung gemäß einem der Ansprüche 1, 2 oder 3, **dadurch gekennzeichnet, dass** die Verbindung mit der Formel (I) dergestalt ist, dass R₁ und/oder R₂ eine Aminosäure sind, deren funktionelle α-Amino- oder α-Carboxylgruppe substituiert ist oder nicht und die aus den folgenden Aminosäuren gewählt wird: Alanin, Cystein, Glycin, Hydroxyprolin, Isoleucin, Leucin, Methionin, Phenylalanin, Prolin, Hydroxyprolin, Serin, Threonin, Tryptophan, Asparagin, Valin, Asparaginsäure, Glutaminsäure, Pyroglutaminsäure, Glutamin, Arginin, Histidin, Lysin.

7. Kosmetische Zusammensetzung gemäß Anspruch 6, **dadurch gekennzeichnet, dass** die Verbindung mit der allgemeinen Formel (I) dergestalt ist, dass R₁ und/oder R₂ Alanin, Leucin, Isoleucin, Phenylalanin, Tryptophan oder Valin sind.

8. Kosmetische Zusammensetzung gemäß Anspruch 6, **dadurch gekennzeichnet, dass** die Verbindung mit der allgemeinen Formel (I) dergestalt ist, dass R₁ und/oder R₂ Methionin, Cystein, Histidin, Asparaginsäure oder Glutaminsäure sind.

9. Kosmetische Zusammensetzung gemäß einem der Ansprüche 1, 2 oder 3, **dadurch gekennzeichnet, dass** die Verbindung mit der Formel (I) dergestalt ist, dass R₁ und/oder R₂ eine Aminosäure darstellen, deren funktionelle α-Amino- oder α-Carboxylgruppe substituiert ist oder nicht und die aus den folgenden Aminosäuren gewählt wird: Citrullin, Homoserin, Norvalin, Ornithin, Penicillamin oder Sarkosin.

10. Kosmetische Zusammensetzung gemäß einem der Ansprüche 1, 2 oder 3, **dadurch gekennzeichnet, dass** die Verbindung mit der Formel (I) dergestalt ist, dass R₁ ein Acyl- oder Acyloxyradikal ist, das eine Isopropyl-, sec-Butyl-, tert-Butyl-, Isobutylgruppe trägt, und/oder R₂ ein Alkoxy- oder Alkylaminoradikal ist, das eine Isopropyl-, n-Butyl-, sec-Butyl, tert-Butyl-, Isobutyl, n-Octyl oder Pivaloyloxymethylgruppe trägt.

11. Kosmetische Zusammensetzung gemäß irgendeinem der Ansprüche 1 bis 10, **dadurch gekennzeichnet, dass** sie zudem ein Antioxidans wie Vitamin E, Ascorbinsäure oder ihre Derivate, oder eine Aminosäure umfasst, die eine Thiolgruppe wie N-Acetyl-cystein besitzt.

12. Kosmetische Zusammensetzung gemäß irgendeinem der Ansprüche 1 bis 11, **dadurch gekennzeichnet, dass** sie zudem ein Pseudodipeptid von der Art jener umfasst, die durch Kopplung von Histamin und einer Aminosäure erzielt wird.

13. Kosmetische Zusammensetzung gemäß einem der Ansprüche 1 bis 12, **dadurch gekennzeichnet, dass** sie zudem einen NO-Synthaseaktivator wie NADPH, Calcium oder jegliche Substanz, die den intrazellulären Calciumgehalt erhöhen kann, enthält.

14. Kosmetische Zusammensetzung gemäß einem der Ansprüche 1 bis 13, **dadurch gekennzeichnet, dass** sie in jeglicher Form vorkommt, die im kosmetischen Bereich üblicherweise verwendet wird, das heißt Creme, Milch, Lotion, Gel, Pflaster oder Spray.

15. Zusammensetzung gemäß einem der vorhergehenden Ansprüche, **dadurch gekennzeichnet, dass** die Verbindung eine endogene Vorstufe von NO° ist.

16. Zusammensetzung gemäß einem der vorhergehenden Ansprüche, **dadurch gekennzeichnet, dass** sie in der Lage ist, durch Freisetzung von NO° auf eine Fettzelle zu wirken.

17. Eine Benutzung von mindestens einer Verbindung, die ein Analogstoff von L-Arginin oder einer seiner Derivate, ein Derivat des L-Arginins oder irgendeines ihrer Salze ist, bei der Herstellung einer pharmazeutischen Zusammensetzung mit topischer Anwendung und schlankmachender Wirkung durch endogene Freisetzung von NO°, wobei die Verbindung die folgende allgemeine Formel (I) aufweist: 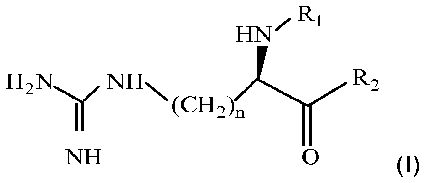 in der:
R₁ ein Wasserstoffatom, eine Hydroxylgruppe, ein Acyl-, Acyloxyradikal oder eine Aminosäure darstellt, deren freie funktionelle α-Aminogruppe substituiert ist oder nicht, gebunden durch eine Peptidbindung,
R₂ ein Alkoxy-, Amino-, Alkylaminoradikal, eine Silyloxygruppe oder eine Aminosäure darstellt, deren funktionelle α-Carboxylgruppe substituiert ist oder nicht, gebunden durch eine Peptidbindung,
und n = 3 oder 4 ist.
